(19) **Europäisches Patentamt European Patent Office Office européen des brevets**

(11) **EP 4 065 237 B1**

(12) **EUROPEAN PATENT SPECIFICATION**

(45) Date of publication and mention
of the grant of the patent:
**24.09.2025 Bulletin 2025/39**

(21) Application number: **20799682.8**

(22) Date of filing: **28.10.2020**

(51) International Patent Classification (IPC):
$A61Q\ 5/12$ [(2006.01)]     $A61K\ 8/39$ [(2006.01)]
$A61K\ 8/37$ [(2006.01)]     $A61K\ 8/34$ [(2006.01)]
$A61K\ 8/06$ [(2006.01)]

(52) Cooperative Patent Classification (CPC):
**A61Q 5/12; A61K 8/062; A61K 8/342; A61K 8/37;
A61K 8/39**

(86) International application number:
**PCT/EP2020/080257**

(87) International publication number:
**WO 2021/104785 (03.06.2021 Gazette 2021/22)**

(54) **A CONDITIONING COMPOSITION**

PFLEGENDE ZUSAMMENSETZUNG

COMPOSITION DE CONDITIONNEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.11.2019 PCT/CN2019/121115**

(43) Date of publication of application:
**05.10.2022 Bulletin 2022/40**

(73) Proprietor: **Beiersdorf AG
22529 Hamburg (DE)**

(72) Inventors:
• **FENG, Ji
Wuhan, 420070 (CN)**
• **ZIA, Caroline
22767 Hamburg (DE)**
• **JIA, Zebao
Wuhan, 430070 (CN)**

(74) Representative: **Beiersdorf AG
Patentabteilung
Beiersdorfstraße 1 - 9
22529 Hamburg (DE)**

(56) References cited:
EP-A2- 0 937 453     WO-A1-2016/090150
US-A- 5 139 770     US-A1- 2009 285 869

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical field

The present invention relates to a cosmetic composition, especially a conditioning composition. The composition has a caring effect and at the same time is easy to be rinsed off.

### Background art

The entire human body, with the exception of the lips, the palms of the hands and the soles of the feet, is covered with hair, albeit for a large part barely visible. Because of many nerve endings at the hair root, hair reacts sensitively to external influences such as wind or touch and is therefore a part of the sense of touch that should not be underestimated. However, nowadays, the most important function of human head hair lies in helping to create the appearance of the person in a characteristic manner. Similarly to the skin, it fulfils a social function because it contributes considerably to interpersonal relations and to the self-esteem of an individual via its outward appearance.

Hair consists of hair shaft and hair root. Hair shaft protrudes freely from the skin, and is a keratinized (dead) section of the hair. Hair shaft represents the actual visible part of the hair, which is continually renewed. Hair root which sticks in the skin is the living part of the hair. Hair shaft consists of three layers: the central part, which is called hair marrow (medulla), regressed in humans, often completely missing; then the marrow, also called cortex; and cuticle as the outermost layer, comprising up to ten horny layers.

Human hair in its freshly grown condition is virtually impossible to improve. The part of the hair in the vicinity of the scalp accordingly has a virtually closed horny layer. In particular, the horny layer, being the external sheath of the hair, and also the inner region below the cuticle are subjected to particular stress by environmental influences.

To keep the hair in a condition resembling freshly grown hair, many consumers apply products with caring and nourishing properties to the hair. Today, the daily routine comprises a step of washing hair and frequently a step of drying the hair using blow-dryer or other drying means, where the hair is exposed to relatively high temperatures. In the long term this step may result in dry hair, which is often only hardly manageable.

Moreover, the hair may be colored, in a permanent or non-permanent way and/or the hair may be permed or uncurled. All these procedures involve the application of sometimes relatively aggressive chemical compounds and together with high temperatures.

These treatments stress the hair resulting in dry, dull, and hardly manageable hair. To improve these undesirable conditions of the hair, many products are on the market. A very famous and often used product is a conditioner, which may be a separate product or included in a shampoo as a 2-in-1 preparation.

Conditioner compositions are known in prior art; only a few will be cited.

WO 2017/036134 A1 describes the effect of lotus flower oil in a hair conditioning composition.

WO 2018/018487 A1 describes the effect of rose hip oil in a hair conditioning composition.

WO 2018/227515 A1 describes the effect of a Cymbidium Grandiflorum Flower Extract in a hair conditioning composition.

WO 2018/095152 A1 discloses a transparent conditioning composition.

The documents US 5139770 A, EP 0937453 A2, WO 2016/090150 A1 and US 2009/285869 A1 disclose various compositions suitable as conditioning rinse-off product.

The main purpose of a conditioner is to provide care for the hair, helping to improve the condition of hair. For this issue, components having a caring effect are included in the conditioner. Such components comprise silicone compounds, natural oils, fatty alcohols, cationic polymers and surfactants, and many more. In many cases it could be shown that the resulting conditioner composition has a caring effect to the hair, but has drawbacks, too. Frequently, said compositions are difficult to be rinsed off, the hair is weighed down and has little volume; sometimes hair even looks greasy.

Consumers do not like products having the above mentioned negative effects.

Therefore, a conditioner should be made available being easily rinsed off and at the same time providing a caring effect to the hair, which is comparable to the one known conditioners show.

### Summary of the invention

Surprisingly, the above mentioned problem could be solved by a conditioner composition, provided as a composition to be rinsed off and containing

- At least one emollient chosen from ester oils and/or dialkylether and/or dialkylcarbonate,
- at least one emulsifier, and
- a combination of fatty alcohols, wherein one or more fatty alcohol(s) having 18 or more carbon atoms is/are contained in a total amount of less than 1.5 % by weight and one or more fatty alcohol(s) having 16 or less carbon atoms is/are contained in a total amount of less than 8.0 % by weight, respectively each one in relation to the total weight of the composition,

wherein the at least one emulsifier is chosen from glyceryl stearate citrate, glyceryl stearate SE, polyglyceryl-3 distearate in admixture with glyceryl stearate citrate, and/or polyglyceryl-3 methyl glucose distearate,

wherein the at least one emulsifier is contained in a total amount of 0.005 to 2.0 % by weight, in relation to the total weight of the composition,

wherein water is contained in an amount of at least 75 % by weight, preferably 85 to 98 % by weight water, more preferably 88 to 95 % by weight water, in relation to the total weight of the composition and

wherein cationic polymers are not contained.

The conditioner composition is an aqueous composition containing at least 75 % by weight water, preferably 85 to 98 % by weight water, more preferably 88 to 95 % by weight water.

The conditioner composition is preferably provided in form of an emulsion, more preferably in form of an O/W emulsion.

In general, it is understood that emulsions are heterogeneous systems, which consist of two liquids which are immiscible or have only limited miscibility with one another and which are usually referred to as phases. In an emulsion, one of the two liquids is dispersed in the form of very fine droplets in the other liquid. The liquids (pure or as solutions) are present in an emulsion in a more or less fine distribution, which is generally of only limited stability.

If the two liquids are water and oil and oil droplets are present in finely distributed form in water, then this is an oil-in-water emulsion (O/W emulsion, e.g. milk). The basic character of an O/W emulsion, exemplified by electrical conductivity, sensory properties, ability of the continuous phase to be stained, is determined by the water. In the case of a water-in-oil emulsion (W/O emulsion, e.g. butter), the basic character is determined by the oil.

To stabilize an emulsion, one or more emulsifier(s) is/are contained in general.

Emulsifiers help to combine two immiscible liquids (for example oil in water) in a way to result in a stable preparation, an emulsion. For that reason emulsifiers have to have an amphiphilic character, the hydrophobic part interacts with the oily or lipid phase and the hydrophilic part interacts with the aqueous phase. By stirring or homogenizing the generated droplets are dispersed in the respective environment, namely aqueous droplets in a lipid environment or lipid droplets in an aqueous environment. Primarily, emulsifiers do not have a detersive, surfactant character. Emulsifiers reduce the interfacial tension between the two phases and, besides reducing the interfacial work, also achieve a stabilization of the emulsion formed. They stabilize the formed emulsion by means of interfacial films, as well as by forming steric or electrical barriers, as a result of which the merging (coalescence) of the emulsified particles is prevented.

HLB values are suitable to characterize emulsifiers, said values specify the hydrophilicity of a given emulsifier. The HLB value may be determined by the following formula:

$$HLB = 20 \times (1 - M_{lipophile}/M),$$

where $M_{lipophile}$ represents the molar mass of the lipophilic fraction of a given emulsifier and M represents the molar mass of the total emulsifier.

In general, emulsifiers with an HLB value up to about 8 are considered to be W/O-emulsifiers. By contrast, O/W-emulsifiers have HLB values of greater than 8 to 15. Substances with HLB values greater than 15 are often referred to as solubilizers.

Suitable emulsifiers may be chosen from:

Ceteareth-12, ceteareth-20, ceteareth-25, ceteareth-3, ceteth-10, ceteth-2, ceteth-20, cetyl phosphate, cetyl stearyl alcohol in combination with PEG-20 stearate, glyceryl stearate citrate, glyceryl stearate in combination with PEG-100 stearate, glyceryl stearate in combination with PEG-30 stearate, glyceryl stearate SE, isosteareth-10, isosteareth-20, isostearyl diglyceryl succinate, laureth-23, laureth-4 phosphate, laureth-4, methyl glucose sesquistearate, PEG-100 stearate, PEG-20 glyceryl stearate, PEG-20 stearate, PEG-20 methyl glucose sesquistearate, PEG-30 stearate, PEG-40 stearate, PEG-45/dodecyl glycol copolymer, PEG-6 caprylic/capric glyceride, polyglyceryl-10 stearate, polyglyceryl-2 laurate, polyglyceryl-2 PEG-4 stearate, polyglyceryl-2 sesquiisostearate, polyglyceryl-3 distearate, polyglyceryl-3 distearate in combination with glyceryl stearate citrate, polyglyceryl-3 methyl glucose distearate, polysorbate 60, potassium cetyl phosphate, sodium cetyl stearyl sulfate, sodium stearoyl glutamate, steareth-10, steareth-2, steareth-20, steareth-21, stearic acid, sucrose polystearate + hydrogenated polyisobutene, triceteareth-4 phosphate, trilaureth-4 phosphate.

Glyceryl stearate citrate, glyceryl stearate SE, polyglyceryl-3 distearate in admixture with glyceryl stearate citrate, and/or polyglyceryl-3 methyl glucose distearate, preferably polyglyceryl-3 methyl glucose distearate is contained in the composition according to the present invention.

Likewise a silicone-based emulsifier may be contained in the composition according to the invention. A suitable emulsifier, having a HLB value of about 2.2 is Lauryl PEG/PPG 18/18 methicone. This emulsifier may be purchased as Dowsil 5200 Formulation Aid from Dow Corning. Further suitable emulsifiers, which may be used, are Bis-PEG/PPG-16/16 PEG/PPG-16/16 Dimethicone (and) Caprylic/Capric Triglyceride (e.g. ABIL® Care 85 from EVONIK), Bis-PEG/PPG-20/5 PEG/PPG-20/5 Dimethicone (and) Methoxy PEG/PPG-25/4 Dimethicone (and) Caprylic/Capric Triglyceride (e.g. ABIL® Care XL 80 from EVONIK), Bis-PEG/PPG-14/14 Dimethicone (and) Dimethicone (e.g. ABIL® EM 97 S from EVONIK), Cetyl PEG/PPG-10/1 Dimethicone (e.g. EMULSIL® S-290 from Innospec Performance Chemicals), Cetyl PEG/PPG-7/3 Dimethicone (e.g. SeraSol SC 82 from KCC Beauty), and PEG/PPG-6/4 Dimethicone (e.g. SeraSol SC 90 from KCC Beauty).

According to the invention, the at least one emulsifier is contained in a total amount of 0.005 to 2.0 % by weight, preferably 0.1 to 1.0% by weight, in relation to the total weight of the composition. If the emulsifier is a single component, the values refer to active content. If the emulsifier is a mixture of two or more components the values refer to the amount of the mixture.

According to the invention the at least one emollient may be chosen from esters of saturated and/or unsaturated, branched and/or unbranched alkane carboxylic acids with a chain length of from 3 to 20 carbon atoms and saturated and/or unsaturated, branched and/or unbranched alcohols with a chain length of from 1 to 20 carbon atoms, and esters of aromatic carboxylic acids and saturated and/or unsaturated, branched and/or unbranched alcohols with a chain length of from 3 to 20 carbon atoms. Such components may be termed ester oils. Examples for suitable ester oils are octyl palmitate, octyl cocoate, octyl isostearate, octyldodecyl myristate, cetearyl isononanoate, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-ethylhexyl stearate, 2-hexyldecyl stearate, 2-octyldodecyl palmitate, stearyl heptanoate, oleyl oleate, oleyl erucate, erucyl oleate, erucyl erucate, tridecyl stearate, tridecyl trimellitate, isoamyl laurate.

It is preferred, if the at least one ester oil is selected from esters of alcohols with a small number of carbon atoms, i.e. having 2 to 8 carbon atoms, for instance such as isopropanol, propylenglycol, neopentyl glycol and isopentyl alcohol and fatty acids, preferably isopentyl alcohol and isopropanol. More preferably the at least one ester oil is chosen from isopropyl myristate and/or isoamyl laurate. Isopropyl myristate may be purchased as Isopropylmyristat (IPM) from BASF and isoamyl laurate may be purchased as Mackaderm Lia from Solvay or as Demofeel Sensolv from Evonik.

If at least one ester oil is contained in the composition of the present invention, the at least one ester oil is contained in a total amount of 0.005 to 5.0 % by weight, preferably 0.1 to 3.0 % by weight, in relation to the total weight of the composition.

According to the invention the at least one emollient may be chosen from at least one dialkylether and/or dialkylcarbonate, preferably dialkylcarbonates, more preferably dicaprylyl carbonate.

If at least one dialkylether and/or dialkylcarbonate is contained in the composition of the present invention, the at least one dialkylether and/or dialkylcarbonate is contained in a total amount of 0.005 to 5 % by weight, preferably 0.1 to 3.0 % by weight, in relation to the total weight of the composition.

In the composition of the present invention a combination of fatty alcohols is contained. Said combination is characterized in comprising one or more fatty alcohol(s) having 18 or more carbon atoms contained in a total amount of less than 1.5 % by weight, especially less than 1.0 % by weight, and one or more fatty alcohol(s) having 16 or less carbon atoms contained in a total amount of less than 8.0 % by weight, preferably less than 6.0 % by weight, more preferably less than 5.0 % by weight, respectively each one in relation to the total weight of the composition.

In general, it is understood that fatty alcohols are straight, long-chained molecules with one hydroxyl group at the first carbon atom. The alkyl residue has 6 to 22 carbon atoms and may have one or multiple double bonds.

It is preferred if stearyl alcohol is one of the alcohols from the group of fatty alcohols having 18 or more carbon atoms.

It is likewise preferred, if myristyl alcohol and/or cetyl alcohol is/are part of the group of fatty alcohols having 16 or less carbon atoms.

It is more preferred, if stearyl alcohol, myristyl alcohol and cetyl alcohol are contained in the composition of the present invention.

It is most preferred, if in the composition of the present invention the combination of fatty alcohols consists of stearyl alcohol, myristyl alcohol and cetyl alcohol.

The compositions of the present invention do contain one or more fatty alcohol(s) having 18 or more carbon atoms. Said alcohol(s) is/are contained in a total amount higher than 0.1 % by weight in relation to the total weight of the composition.

The compositions of the present invention do contain one or more fatty alcohol(s) having 16 or less carbon atoms. Said alcohol(s) is/are contained in a total amount higher than 0.1 % by weight in relation to the total weight of the composition.

In addition to the above mentioned emollient, additionally one or more moisturizer(s) may be contained in the composition according to the invention.

Advantageously said moisturizers contain hydrophilic residues, which may lead to substances being hygroscopic. Hygroscopic substances bind water and therefore may provide moisture. The hygroscopic quality is due to hydrophilic substituents of the molecule, in many cases hydroxyl groups, but other functional groups, such as amine or carboxyl groups can fulfil the same function. Examples of moisturizer are propylene glycol, hexylene glycol, and butylene glycol, glyceryl triacetate, glycerol, sorbitol, xylitol, maltitol, poly dextrose, hydrogenated starch hydrolysate, urea, Aloe vera gel, alpha hydroxy acids such as lactic acid, honey, and/or polyethylene glycols such as PEG-4, PEG- 6, PEG-7, PEG-8, PEG-9, PEG-10, PEG-12, PEG-14, PEG-16, PEG-18, PEG-20, PEG-32, PEG-40, PEG-55, PEG-60, PEG-75, PEG-90, PEG-100, PEG-135, PEG-150, PEG-154, PEG-180, PEG-200, PEG-240. It is preferred, if at least one moisturizer having a smaller molecular weight, especially a molecular weight below 250 g/mol and at least one moisturizer having a higher molecular weight, especially a molecular weight above 500 g/mol, further especially above 1000 g/mol are contained in the composition of the present invention. It is more preferred, if glycerine and PEG-150 are contained in the composition of the present invention. It is even more preferred, if in relation to moisturizer only glycerine and PEG-150 are contained in the composition of the present invention. In case that in relation to moisturizers only glycerine and PEG-150 are contained in the composition of the present invention, it is most preferred, if glycerine is contained in an amount of 1.5 to 2.5 % by weight, especially 2 % by weight, in relation to the total weight of the composition, and PEG-150 is contained in an amount of 0.1 to 0.2 % by weight, especially 0.15 % by weight, in relation to the total weight of the composition.

In the composition of the present invention one or more moisturizer is/are contained in a total amount of 0.001 to 10 % by weight, preferably 0.005 to 6.0 % by weight, in relation to the total weight of the composition.

Additionally, at least one cellulose compound may be contained in the composition of the present invention. Cellulose compounds also comprise cellulose derived cellulose compounds. Cellulose compounds are characterized by a chemical structure containing links of glucose residues, which are β-1,4-bonds. In addition to unsubstituted celluloses, cellulose derivatives may be anionic, amphoteric or nonionic. Cellulose ethers, cellulose esters and cellulose ether esters have to be distinguished from these derivatives.

According to the present invention cellulose compounds are selected from the non-ionic cellulose ethers. Nonionic cellulose ethers include alkyl celluloses, such as methyl celluloses and ethyl celluloses; hydroxyalkyl celluloses, such as hydroxymethyl celluloses, hydroxyethyl celluloses and hydroxypropyl celluloses; mixed hydroxyalkyl alkyl celluloses, also known as mixed cellulose ethers.

According to the present invention it is preferred, if at least one hydroxyethyl cellulose is contained in the composition according to the present invention. A suitable hydroxyethyl cellulose may be obtained from CG Chemikalien under the trade name Natrosol 250 HHX pharm, for example.

The at least one cellulose compound, especially the at least one hydroxyethyl cellulose is contained in a total amount of 0.08 % to 1.0 % by weight, preferably from 0.1 % to 0.6 % by weight, in relation to the total weight of the composition and in relation to the active content.

Likewise additionally at least one guar compound may be contained in the composition of the present invention. Guar gum, also called guaran, is a polysaccharide built from galactose and mannose residues, wherein the mannose residues are β 1,4 linked forming the backbone of the molecule. Galactose residues are linked via β 1,6 glycosidic linkage at every second mannose resulting in a branching of the molecule. In addition to unsubstituted guar gums, guar gum derivatives may be contained, e.g. hydroxypropyl guar.

According to the invention, additionally at least one cationic surfactant may be contained in the composition of the present invention. Cationic surfactants are preferably those which have conditioning qualities. More preferably, the cationic surfactants are quaternary ammonium salts. It is still more preferred that the quaternary ammonium salts contain nitrogen atoms having three methyl groups as substituents, whereas the fourth substituent is a long-chained alkyl residue which may be straight, branched, substituted or non-substituted. It is even still more preferred, if the alkyl residue is a straight, non-substituted hydrocarbon group containing 12 to 28 carbon atoms. It is even further still more preferred if the alkyl residue contains 18 to 24 carbon atoms. The corresponding anion may be selected from the group of chloride, bromide and methosulfate. It is most preferred to use behentrimonium chloride, which may be purchased as Genamin BTLF from Clariant.

If at least one cationic surfactant is contained in the composition of the present invention, the at least one cationic surfactant is contained in an amount of 0.1 to 3.0 % by weight, more preferably 0.2 to 2.5 % by weight, in relation to the total weight of the composition. The values are referring to the active content of the cationic surfactants.

According to the invention it is the composition of the present invention does not contain any cationic polymer. Cationic polymers are compounds consisting of a high number of repeating monomers, which may be of the same chemical structure or which may be of differing chemical structures. Cationic polymers do have a positive net charge, which is frequently due to nitrogen atoms. These nitrogen atoms may have a persisting positive charge, being independent of the pH value (ammonium group) or a pH dependent positive charge (amine group).

Additionally, the composition according to the present invention may comprise at least one preservative. Suitable preservatives are those ones, which are allowed and suitable for cosmetic compositions. However, it is preferred to use of Benzyl Alcohol, Methylisothiazolinone, Methylchloroisothiazolinone, Ethylparaben, Methylparaben, Phenoxyethanol, and mixtures thereof.

The preferred preservatives may be used alone or in combination. If at least one preservative is comprised in the composition of the present invention, the at least one preservative is contained in a total amount of 0.1 to 1.0 % by weight, preferably 0.4 to 0.8 % by weight, in relation to the total weight of the composition. The values are referring to the active content of the preservatives.

The pH value of the composition according to the present invention may be adjusted by all means suitable for cosmetic preparations. It is preferred, if the composition has a pH value of 3.0 to 7.0, especially 3.1 to 6.8.

The composition of the present invention may be prepared by any technique known or effective to prepare a hair conditioning composition. The process to prepare the composition of the present invention comprises conventional formulating and mixing techniques.

Specifically, compositions of the present invention are preferably prepared using the following procedure:
For manufacturing a batch of one of the compositions mentioned in the present invention, an IKA RW20 digital or IKA Eurostar stir equipment and a stirrer, e.g. a paddle stirrer is used.

- Mix water, moisturizers, and cationic surfactant, this mixture being the water phase, stir this phase and while heating to 70- 85°C in a beaker until all components are dissolved.
- Mix fatty alcohols, emollients, cellulose and emulsifiers, this mixture being the oily phase, stir this phase while heating to 70 - 85°C in a beaker until all components are dissolved or dispersed.
- Combine water phase and oily phase, mix and homogenize until a uniform composition is achieved.

To evaluate the property of a quick rinsing composition an analysis of market products was conducted (examples, table 4). Different products were analyzed in relation to the time necessary to rinse off the respective products. These tests were conducted by an expert hair panel using hair tresses 10g strands from Kerling, bleached for 2 hours. 2g of the product to be tested was evenly distributed on hair by hands for 30 seconds. Under a constant water stream of $5 \pm 0.5$ l/min at 38°C, the rinsing time, measured in seconds, was evaluated by experts via a characteristic sensory change. A rinsing-off time below 10 seconds is evaluated as a quick rinsing time. A rinsing-off time of more than 14 seconds is a slow rinsing time; the consumer will evaluate such a product as being a bad rinsable one.

Two products, Garnier Wahre Schätze Honig and Gliss Kur are described by the property of easy rinsing off. As shown in table 4, for rinsing off these two products only 8 and 7 seconds are necessary. Hence, to provide a conditioner composition, which may be described as a fast rinsing composition, the rinsing off should be achieved in the same time as shown for the market products.

In a first step, the composition and amount of fatty alcohols were analyzed. It was realized that a combination of fatty alcohols is advantageously. Said combination contains one or more fatty alcohol(s) having 18 or more carbon atoms in a total amount of less than 1.0 % by weight, in relation to the total weight of the composition and one or more fatty alcohol(s) having 16 or less carbon atoms contained in a total amount of less than 5.0 % by weight, in relation to the total weight of the composition.

In a second step, the influence of the emollient was determined. In a comparison the ester oils isopropyl myristate and isoamyl laurate were compared to a raw material, which is called undecane/tridecane (examples, table 1). Undecane/tridecane is a natural-based emollient, which is prepared from 100% renewable components. Undecane/-tridecane is described as a possible substitute for cyclomethicones. However, the composition containing either isopropyl myristate or isoamyl laurate as well as the one containing isopropyl alcohol and isoamyl laurate are significantly easier to rinse off than the composition containing undecane/tridecane.

In a further step, additional emollients were tested, namely dicaprylyl carbonate and coco-caprylate/caprate and again undecane/tridecane (examples, table 2). The rinsing times necessary to rinse off the respective products show, that isoamyl laurate is the best emollient in relation to fast rinsing times. But products containing dicaprylyl carbonate also lead to quick rinsing times. The products containing coco-caprylate/caprate and undecane/tridecane result in slow rinsing times.

Summing up, esteroils, exemplified by isopropyl myristate and isoamyl laurate and dialkyl carbonates, exemplified by dicaprylyl carbonate, are emollients, which may advantageously by used, when conditioner compositions shall be provided with fast rinsing times.

In a last step, the influence of the emulsifier was analyzed. Several O/W emulsifiers, namely glyceryl stearate SE, glyceryl stearyl citrate, polyglyceryl-3 methylglucose distearate and polyglyceryl-3 distearate in admixture with glyceryl stearate citrate, were included in conditioner compositions (examples, table 3). Furthermore, two W/O emulsifiers were used, namely glyceryl stearate and a silicone-based emulsifier, lauryl PEG/PPG-18/18 methicone. With the exception of the W/O emulsifier glyceryl stearate, the use of all the other emulsifiers leads to conditioner compositions with quick rinsing times.

As a conditioning composition must have an obvious conditioning effect, gliding and combing properties of the hair were determined.

Hair tresses (in each case 10g strands from Kerling, bleached for 2hours) were used. 2g of the respective products to be tested were evenly distributed on hair by hands for 30seconds; now the property "gliding" was evaluated on a scale from 1 to 10, "1" being blunt and "10" being very slippery. After the rinsing off the respective products using the same procedure as described above, the hair was combed twice and the resistance was estimated on a scale from 1 to 10, "1" being not combable, and "10" being very easily combable without any resistance. It became apparent that the compositions containing the emulsifier lauryl PEG/PPG-18/18 methicone or polyglyceryl-3 distearate in admixture with glyceryl stearate citrate provided a good conditioning effect to the hair.

As shown the composition according to the invention could solve the problem underlying the present invention.

**Examples**

The examples below are intended to illustrate the present invention without limiting it. The numerical values in the examples are percentages by weight, based on the total weight of the particular compositions, given as active content.

Table 1: Evaluation of the emollient in examples 1-4 (not according to the invention)

| All the values are given as wt% and are referring to active content, if not denoted oth erwise. | | | | |
|---|---|---|---|---|
| **INCI** | **1** | **2** | **3** | **4** |
| Aqua | 91.22 | 91.22 | 91.22 | 89.04 |
| Myristyl Alcohol | 1 | 1 | 1 | 0.5 |
| Cetyl Alcohol | 3 | 3 | 3 | 3 |
| Isopropyl Myristate | | 0.5 | | 0.5 |
| Phenoxyethanol | 0.5 | 0.5 | 0.5 | 0.5 |
| PEG-150 | 0.2 | 0.2 | 0.2 | 0.2 |
| Glycerin | | | | 2 |
| Lauryl PEG/PPG-18/18 Methicone | 0.5 | 0.5 | 0.5 | 0.5 |
| Hydroxyethylcellulose | 0.5 | 0.5 | 0.5 | 0.6 |
| Stearyl Alcohol | 0.6 | 0.6 | 0.6 | 0.6 |
| Perfume | 0.7 | 0.7 | 0.7 | 0.7 |
| Behentrimonium Chloride | 1.28 | 1.28 | 1.28 | 1.36 |
| Undecane, Tridecane | | | 0.5 | |
| Isoamyl Laurate | 0.5 | | | 0.5 |
| **Rinsing time** (s) | 8 | 6 - 8 | 18 | 8 |

Table 2: Further evaluation of the emollient in examples 5-8 (not according to the invention)

| All the values given are referring to active content, if not denoted otherwise. | | | | |
|---|---|---|---|---|
| **INCI** | **5** | **6** | **7** | **8** |
| | m [g] | m [g] | m [g] | m [g] |
| Aqua | 93.35 | 93.35 | 93.35 | 92.746 |
| Cetyl Alcohol | 3 | 3 | 3 | 3.03 |
| Phenoxyethanol | 0.5 | 0.5 | 0.5 | 0.51 |
| Lauryl PEG/PPG-18/18 Methicone | 0.4 | 0.4 | 0.4 | 0.4 |
| Stearyl Alcohol | 1 | 1 | 1 | 1 |

(continued)

| All the values given are referring to active content, if not denoted otherwise. | | | | |
|---|---|---|---|---|
| **INCI** | **5** | **6** | **7** | **8** |
| | m [g] | m [g] | m [g] | m [g] |
| Dicaprylyl Carbonate | | 1 | | |
| Behentrimonium Chloride | 0.5 | 0.5 | 0.5 | 1.064 |
| Undecane, Tridecane | | | 1 | |
| Parfum | 0.25 | 0.25 | 0.25 | 0.25 |
| Isoamyl Laurate | 1 | | | |
| Coco-Caprylate/Caprate | | | | 1 |
| **Rinsing time** (s) | 8 - 9 | 12 | 20 | 17 |

Table 3: Evaluation of the emulsifier in examples 9-14

| All the values given are referring to active content, if not denoted otherwise. The examples 9, 10, 12 and 13 are not according to the invention. | | | | | | |
|---|---|---|---|---|---|---|
| **INCI** | **9** | **10** | **11** | **12** | **13** | **14** |
| | m [g] | m [g] | m [g] | m [g] | m [g] | m [g] |
| Aqua | Ad 100 | Ad 100 | Ad 100 | Ad 100 | Ad 100 | Ad 100 |
| Glyceryl Stearate | | | | | 0.4 | |
| Myristyl Alcohol | 1.3 | 1 | 1 | 1 | 1 | |
| Cetyl Alcohol | 3 | 3 | 3 | 3 | 3 | 3 |
| Isopropyl Myristate | 0.5 | 0.5 | 0.5 | 0.5 | 0.5 | 0,6 |
| Glyceryl Stearate SE | | | | 0.4 | | |
| Phenoxyethanol | 0.5 | 0.5 | 0.5 | 0.5 | 0.5 | 0,5 |
| PEG-150 | 0.15 | 0.15 | 0.15 | 0.15 | 0.15 | 0,15 |
| Glycerin | | 2 | 2 | 2 | 2 | 2 |
| Lauryl PEG/PPG-18/18 Methicone | 0.5 | | | | | |
| Hydroxyethylcellulose | 0.5 | 0.5 | 0.5 | 0.5 | 0.5 | 0,6 |
| Stearyl Alcohol | 0.6 | 0.6 | 0.6 | 0.6 | 0.6 | 0,6 |
| Glyceryl Stearate Citrate | | 0.4 | | | | |
| Behentrimonium Chloride | 1.2 | 1.05 | 1.05 | 1.05 | 1.05 | 1.4 |
| Perfume | 0.7 | 0.7 | 0.7 | 0.7 | 0.7 | 0,7 |
| Polyglyceryl-3 Distearate/Glyceryl Stearate Citrate | | | 0.4 | | | |
| Polygyceryl-3 Methylglycose Distearate | | | | | | 0,4 |
| | | | | | | |
| **Rinsing time** (s) | 8-10 | 8 | 11 | 12 | > 17 | |
| **Gliding** | yes | no | yes | no | yes | |
| **Combing** | yes | no | yes | no | yes | |

Table 4: Evaluation of competitor products

| | Rinsing time (s) |
|---|---|
| | |

(continued)

|  | Rinsing time (s) |
|---|---|
| Nivea Hairmilk normal | > 13 |
| Pantene | > 13 |
| Fructis | > 13 |
| SLEK | > 17 |
| Garnier Wahre Schätze Honig | 8 |
| Gliss Kur | 7 |

Nivea Hairmilk normal, ingredients according to INCI listing: Aqua, Cetyl Alcohol, Myristyl Alcohol, Dimethicone, Stearamidopropyl Dimethylamine, Lanolin Alcohol (Eucerit®), Hydrolyzed Milk Protein, Panthenol, Silicone Quaternium-18, Guar Hydroxypropyltrimonium Chloride, Coco-Betaine, Trideceth-6, Sodium Chloride, Trideceth-12, C12-15 Pareth-3, Cocamidopropyl Betaine, Lactic Acid, Phenoxyethanol, Limonene, Linalool, Geraniol, Benzyl Alcohol, Citronellol, Alpha-Isomethyl Ionone, Perfume.

Pantene, ingredients according to INCI listing: Aqua, Cetyl Alcohol, Stearamidopropyl Dimethylamine, Glycerin, Stearyl Alcohol, Glutamic Acid, Bis-Aminopropyl Dimethicone, Citric Acid, Parfum, Benzyl Alcohol, Disodium EDTA, Phenoxyethanol, Panthenol, Panthenyl Ethyl Ether, Histidine, Hexyl Cinnamal, Hydroxycitronellal, Magnesium Nitrate, Sodium Chloride, Methylchloroisothiazolinone, Magnesium Chloride, Methylisothiazolinone.

Fructis 3 Oil Repair, ingredients according to INCI listing: Aqua, Cetearyl Alcohol, Behentrimonium Chloride, Cetyl Esters, Niacinamide, Saccharum Officinarum Extract / Sugar Cane Extract, Cocos Nucifera Oil / Ccoconut Oil, Tocopherol, Hydroxypropyltrimonium Lemon Protein, Olea Europeae Oil / Olive Fruit Oil, Phenoxyethanol, Trideceth-6, Chlorhexidine Digluconate, Helianthus Annuus Seed Oil / Sunflower Seed Oil, Limonene, Camellia Sinensis Leaf Extract, Linalool, Benzyl Salicylate, Benzyl Alcohol, Amodimethicone, Isopropyl Alcohol, Pyrus Malus Fruit Extract / Apple Fruit Extract, Pyridoxine HCl, Persea Gratissima Oil / Avocado Oil, Rosmarimus Officinalis Laef Extract / Rosemary Leaf Extract, Citric Acid, Butylphenyl Methylpropional, Cetrimonium Chloride, Bytospermum Parkii Butter / Shea Butter, Citrus Limon Peel Extract / Lemon Peel Extract, Leuconostoc/ Radish Root Ferment Filtrate, Hexyl Cinnamal, Parfum / Fragrance.

SLEK Hot Oil, ingredients according to INCI listing: Aqua, Stearyl Alcohol, Dimethicone, Behentrimonium Chloride, Cetyl Alcohol, Phenoxyethanol, Aminoethylaminopropyl Dimethicone, Isopropyl Alcohol, Amodimethicone, PPG-3 Caprylyl Ether, Panthenol, Linalool, Vitis Vinifera Seed Oil, Trideceth-10, Cetrimonium Chloride, Citronellol, Cyclomethicone, Limonene, Geraniol, Coumarin, Hydrogen Peroxide, Acetic Acid, Methyl Benzoate, Citric Acid, BHT, Sodium Benzoate, Soluble Collagen, Citral, Benzyl Alcohol, Parfum.

Garnier Wahre Schätze Honig, ingredients according to INCI listing: Aqua/Water, Cetyl alcohol, PEG-180, CI 19140/Yellow 5, CI 15985/Yellow 6, Elaeis Guineensis Oil/Palm Oil, Hydroxyethylcellulose, Dodecene, Mel/Honey, Chlorhexidine Digluconate, Poloxamer 407, Limonene, Benzyl Alcohol, Benzyl Salicylate, Isopropyl Myristate, 2-Oleamido-1,3-Octadecandiol, Propolis Extract, Myristyl Alcohol, Cetrimonium Chloride, Cetyl Esters, BHT, Citric Acid, Lauryl PEG/PPG-18/18 Methicone, Coumarin, Royal Jelly, Perfume/Fragrance (F.I.L.C164325/2).

Gliss Kur Total Repair Conditioner, ingredients according to INCI listing: Aqua, Cetearyl Alcohol, Quaternium-87, Stearamidopropyl Dimethylamine, Dimethicone, Citric Acid, Distearoylethyl Hydroxyethylmonium Methosulfate, Isopropyl Myristate, Hydrolyzed Keratin, Glycerin, Propylene Glycol, Polyquaternium-10, Panax Ginseng Root Extract, Arginine, Arctium Majus Root Extract, Acetyl Tyrosine, Hydrolyzed Soy Protein, Polyquaternium-11, PEG-12 Dimethicone, Niacinamide, Ornithine HCl, Citrulline, Biotin, Panthenol, Cocodimonium Hydroxypropyl Hydrolyzed Keratin, Phenoxyethanol, Sodium Methylparaben, Parfum, Dimethiconol.

## Claims

1.    A conditioning composition, being rinsed off and containing

- at least one emollient chosen from ester oils and/or dialkylether and/or dialkylcarbonate,
- at least one emulsifier
- a combination of fatty alcohols, wherein one or more fatty alcohol(s) having 18 or more carbon atoms is/are contained in a total amount of less than 1.5 % by weight and one or more fatty alcohol(s) having 16 or less carbon atoms is/are contained in a total amount of less than 8.0 % by weight, respectively each one in relation to the total weight of the composition,
wherein the at least one emulsifier is chosen from glyceryl stearate citrate, glyceryl stearate SE, polyglyceryl-3 distearate in admixture with glyceryl stearate citrate, and/or polyglyceryl-3 methyl glucose distearate,
wherein the at least one emulsifier is contained in a total amount of 0.005 to 2.0 % by weight, in relation to the total weight of the composition,
wherein water is contained in an amount of at least 75 % by weight, preferably 85 to 98 % by weight water, more prefer ably 88 to 95 % by weight water, in relation to the total weight of the composition and
wherein cationic polymers are not contained.

2.  The composition according to claim 1 **characterized in that** the composition is an emulsion, preferably an O/W emulsion.

3.  Composition according to claim 1 or 2 **characterized in that** the at least one emulsifier is polyglyceryl-3 distearate in admixture with glyceryl stearate citrate.

4.  The composition according to any of the preceding claims **characterized in that** additionally at least one silicone-based emulsifier is contained, preferably lauryl PEG/PPG 18/18 methicone.

5.  The composition according to any of the preceding claims **characterized in that** the at least one emulsifier is contained in a total amount of 0.1 to 1.0 % by weight, in relation to the total weight of the composition.

6.  The composition according to any of the preceding claims **characterized in that** the at least one emollient chosen from at least one ester oil is selected from octyl palmitate, octyl cocoate, octyl isostearate, octyldodecyl myristate, cetearyl isononanoate, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-ethylhexyl stearate, 2-hexyldecyl stearate, 2-octyldodecyl palmitate, stearyl heptanoate, oleyl oleate, oleyl erucate, erucyl oleate, erucyl erucate, tridecyl stearate, tridecyl trimellitate, isoamyl laurate, preferably from isopropyl myristate and/or isoamyl laurate.

7.  The composition according to claim 6 **characterized in that** the at least one ester oil is contained in a total amount of 0.005 to 5.0% by weight, preferably 0.1 to 3.0 % by weight, in relation to the total weight of the composition.

8.  The composition according to any of the preceding claims **characterized in that** the at least one emollient is chosen from at least one dialkylether and/or dialkylcarbonate, preferably from at least one dialkylcarbonate, more preferably dicaprylyl carbonate.

9.  The composition according to claim 8 **characterized in that** the at least one dialkylether and/or dialkylcarbonate is contained in a total amount of 0.005 to 5.0 % by weight, preferably 0.1 to 3.0 % by weight, in relation to the total weight of the composition.

10.  The composition according to any of the preceding claims **characterized in that** one of the fatty alcohol(s) having 18 or more carbon atoms is stearyl alcohol, preferably the fatty alcohol having 18 or more carbon atoms is stearyl alcohol.

11.  The composition according to any of the preceding claims **characterized in that** myristyl alcohol and/or cetyl alcohol is/are part of the group of fatty alcohols having 16 or less carbon atoms, preferably the fatty alcohols having 16 or less carbon atoms are myristyl alcohol and cetyl alcohol.

12.  The composition according to any of the preceding claims **characterized in that** the at least one of the fatty alcohol(s) having 18 or more carbon atoms is contained in a total amount of less than 1 % by weight, in relation to the total weight of the composition.

13.  The composition according to any of the preceding claims **characterized in that** the at least one of the fatty alcohol(s) having 18 or more carbon atoms is contained in a total amount of higher than 0.1 % by weight, in relation to the total weight of the composition.

14.  The composition according to any of the preceding claims **characterized in that** the at least one fatty alcohol(s) having 16 or less carbon atoms is contained in a total amount of less than 6.0 % by weight, preferably less than 5.0 % by weight, in relation to the total weight of the composition.

**15.** The composition according to any of the preceding claims **characterized in that** the at least one of the fatty alcohol(s) having 16 or less carbon atoms is contained in a total amount of higher than 0.1 % by weight, in relation to the total weight of the composition.

**16.** The composition according to any of the preceding claims **characterized in that** additionally one or more moisturizer(s) are contained, preferably chosen from propylene glycol, hexylene glycol, and butylene glycol, glyceryl triacetate, glycerol, sorbitol, xylitol, maltitol, poly dextrose, hydrogenated starch hydrolysate, urea, Aloe vera gel, alpha hydroxy acids such as lactic acid, honey, and/or polyethylene glycols such as PEG-4, PEG- 6, PEG-7, PEG-8, PEG-9, PEG-10, PEG-12, PEG-14, PEG-16, PEG-18, PEG- 20, PEG-32, PEG-40, PEG-55, PEG-60, PEG-75, PEG-90, PEG-100, PEG-135, PEG- 150, PEG-154, PEG-180, PEG-200, PEG-240, more preferably chosen from glycerine and PEG-150.

**17.** The composition according to claim 16 **characterized in that** the at least one moisturizer is contained in a total amount of 0.001 to 10 % by weight, preferably 0.005 to 6.0 % by weight, in relation to the total weight of the composition.

**18.** The composition according to any of the preceding claims **characterized in that** additionally at least one cellulose compound is contained, preferably at least one hydroxy-alkyl cellulose, more preferably at least one hydroxyethyl cellulose.

**19.** The composition according to claim 18 **characterized in that** the at least one cellulose compound is contained in a total amount of 0.08 % to 1.0 % by weight, preferably from 0.1 % to 0.6 % by weight, in relation to the total weight of the composition.

**20.** The composition according to any of the preceding claims **characterized in that** additionally at least one cationic surfactant is contained, preferably at least one quaternary ammonium salt, more preferably behentrimonium chloride.

**21.** The composition according to claim 20 **characterized in that** the at least one cationic surfactant is contained in a total amount of 0.1 to 3.0 % by weight, preferably 0.2 to 2.5 % by weight, in relation to the total weight of the composition.

## Patentansprüche

**1.** Konditionierende Zusammensetzung, die ausgespült wird und Folgendes enthält:

- mindestens ein Emolliens, ausgewählt aus Esterölen und/oder Dialkylether und/oder Dialkylcarbonat,
- mindestens einen Emulgator
- eine Kombination von Fettalkoholen, wobei ein Fettalkohol oder mehrere Fettalkohole mit 18 oder mehr Kohlenstoffatomen in einer Gesamtmenge von weniger als 1,5 Gew.-% enthalten ist/sind und ein Fettalkohol oder mehrere Fettalkohole mit 16 oder weniger Kohlenstoffatomen in einer Gesamtmenge von weniger als 8,0 Gew.-% enthalten ist/sind, jeweils bezogen auf das Gesamtgewicht der Zusammensetzung,
wobei der mindestens eine Emulgator aus Glycerylstearatcitrat, Glycerylstearat SE, Polyglyceryl-3-distearat in Abmischung mit Glycerylstearatcitrat und/oder Polyglyceryl-3-methylglucosedistearat ausgewählt ist,
wobei der mindestens eine Emulgator in einer Gesamtmenge von 0,005 bis 2,0 Gew.-%, bezogen auf das Gesamtgewicht der Zusammensetzung, enthalten ist,
wobei Wasser in einer Menge von mindestens 75 Gew.-%, vorzugsweise 85 bis 98 Gew.-% Wasser, weiter bevorzugt 88 bis 95 Gew.-% Wasser, bezogen auf das Gesamtgewicht der Zusammensetzung, enthalten ist und wobei kationische Polymere nicht enthalten sind.

**2.** Zusammensetzung nach Anspruch 1, **dadurch gekennzeichnet, dass** es sich bei der Zusammensetzung um eine Emulsion, vorzugsweise eine O/W-Emulsion, handelt.

**3.** Zusammensetzung nach Anspruch 1 oder 2, **dadurch gekennzeichnet, dass** es sich bei dem mindestens einen Emulgator um Polyglyceryl-3-distearat in Abmischung mit Glycerylstearatcitrat handelt.

**4.** Zusammensetzung nach einem der vorhergehenden Ansprüche, **dadurch gekennzeichnet, dass** zusätzlich mindestens ein Emulgator auf Silikonbasis enthalten ist, vorzugsweise Lauryl PEG/PPG 18/18 Methicone.

**5.** Zusammensetzung nach einem der vorhergehenden Ansprüche, **dadurch gekennzeichnet, dass** der mindestens eine Emulgator in einer Menge von 0,1 bis 1,0 Gew.-%, bezogen auf das Gesamtgewicht der Zusammensetzung, enthalten ist.

6. Zusammensetzung nach einem der vorhergehenden Ansprüche, **dadurch gekennzeichnet, dass** das mindestens eine aus mindestens einem Esteröl ausgewählte Emolliens aus Octylpalmitat, Octylcocoat, Octylisostearat, Octyldodecylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Ethylhexylstearat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, Isoamyllaurat, weiter bevorzugt aus Isopropylmyristat und/oder Isoamyllaurat, ausgewählt ist.

7. Zusammensetzung nach Anspruch 6, **dadurch gekennzeichnet, dass** das mindestens eine Esteröl in einer Gesamtmenge von 0,005 bis 5,0 Gew.-%, vorzugsweise 0,1 bis 3,0 Gew.-%, bezogen auf das Gesamtgewicht der Zusammensetzung, enthalten ist.

8. Zusammensetzung nach einem der vorhergehenden Ansprüche, **dadurch gekennzeichnet, dass** das mindestens eine Emolliens aus mindestens einem Dialkylether und/oder Dialkylcarbonat, vorzugsweise mindestens einem Dialkylcarbonat, weiter bevorzugt Dicaprylylcarbonat, ausgewählt ist.

9. Zusammensetzung nach Anspruch 8, **dadurch gekennzeichnet, dass** der/das mindestens eine Dialkylether und/oder Dialkylcarbonat in einer Gesamtmenge von 0,005 bis 5,0 Gew.-%, vorzugsweise 0,1 bis 3,0 Gew.-%, bezogen auf das Gesamtgewicht der Zusammensetzung, enthalten ist.

10. Zusammensetzung nach einem der vorhergehenden Ansprüche, **dadurch gekennzeichnet, dass** es sich bei einem der Fettalkohole mit 18 oder mehr Kohlenstoffatomen um Stearylalkohol handelt und es sich vorzugsweise bei dem Fettalkohol mit 18 oder mehr Kohlenstoffatomen um Stearylalkohol handelt.

11. Zusammensetzung nach einem der vorhergehenden Ansprüche, **dadurch gekennzeichnet, dass** Myristylalkohol und/oder Cetylalkohol Teil der Gruppe von Fettalkoholen mit 16 oder weniger Kohlenstoffatomen ist/sind und es sich vorzugsweise bei dem Fettalkohol mit 16 oder weniger Kohlenstoffatomen um Myristylalkohol und Cetylalkohol handelt.

12. Zusammensetzung nach einem der vorhergehenden Ansprüche, **dadurch gekennzeichnet, dass** der mindestens eine der Fettalkohole mit 18 oder mehr Kohlenstoffatomen in einer Gesamtmenge von weniger als 1 Gew.-%, bezogen auf das Gesamtgewicht der Zusammensetzung, enthalten ist.

13. Zusammensetzung nach einem der vorhergehenden Ansprüche, **dadurch gekennzeichnet, dass** der mindestens eine der Fettalkohole mit 18 oder mehr Kohlenstoffatomen in einer Gesamtmenge von mehr als 0,1 Gew.-%, bezogen auf das Gesamtgewicht der Zusammensetzung, enthalten ist.

14. Zusammensetzung nach einem der vorhergehenden Ansprüche, **dadurch gekennzeichnet, dass** der mindestens eine der Fettalkohole mit 16 oder weniger Kohlenstoffatomen in einer Gesamtmenge von weniger als 6,0 Gew.-%, vorzugsweise weniger als 5,0 Gew.-%, bezogen auf das Gesamtgewicht der Zusammensetzung, enthalten ist.

15. Zusammensetzung nach einem der vorhergehenden Ansprüche, **dadurch gekennzeichnet, dass** der mindestens eine der Fettalkohole mit 16 oder weniger Kohlenstoffatomen in einer Gesamtmenge von mehr als 0,1 Gew.-%, bezogen auf das Gesamtgewicht der Zusammensetzung, enthalten ist.

16. Zusammensetzung nach einem der vorhergehenden Ansprüche, **dadurch gekennzeichnet, dass** zusätzlich ein oder mehrere Feuchthaltemittel enthalten sind, die vorzugsweise aus Propylenglykol, Hexylenglykol und Butylenglykol, Glyceryltriacetat, Glycerin, Sorbitol, Xylitol, Maltitol, Polydextrose, hydriertem Stärkehydrolysat, Harnstoff, Aloe-vera-Gel, alpha-Hydroxysäuren wie Milchsäure, Honig und/oder Polyethylenglykolen wie PEG-4, PEG-6, PEG-7, PEG-8, PEG-9, PEG-10, PEG-12, PEG-14, PEG-16, PEG-18, PEG-20, PEG-32, PEG-40, PEG-55, PEG-60, PEG-75, PEG-90, PEG-100, PEG-135, PEG-150, PEG-154, PEG-180, PEG-200, PEG-240 ausgewählt sind und weiter bevorzugt aus Glycerin und PEG-150 ausgewählt sind.

17. Zusammensetzung nach Anspruch 16, **dadurch gekennzeichnet, dass** das mindestens eine Feuchthaltemittel in einer Gesamtmenge von 0,001 bis 10 Gew.-%, vorzugsweise 0,005 bis 6,0 Gew.-%, bezogen auf das Gesamtgewicht der Zusammensetzung, enthalten ist.

18. Zusammensetzung nach einem der vorhergehenden Ansprüche, **dadurch gekennzeichnet, dass** zusätzlich mindestens eine Celluloseverbindung enthalten ist, vorzugsweise mindestens ein Hydroxyalkylcellulose, weiter bevorzugt mindestens eine Hydroxyethylcellulose.

19. Zusammensetzung nach Anspruch 18, **dadurch gekennzeichnet, dass** die mindestens eine Celluloseverbindung in einer Gesamtmenge von 0,08 bis 1,0 Gew.-%, vorzugsweise 0,1 bis 0,6 Gew.-%, bezogen auf das Gesamtgewicht der Zusammensetzung, enthalten ist.

20. Zusammensetzung nach einem der vorhergehenden Ansprüche, **dadurch gekennzeichnet, dass** zusätzlich mindestens ein kationisches Tensid enthalten ist, vorzugsweise mindestens ein quartäres Ammoniumsalz, weiter bevorzugt Behentrimoniumchlorid.

21. Zusammensetzung nach Anspruch 20, **dadurch gekennzeichnet, dass** das mindestens eine kationische Tensid in einer Gesamtmenge von 0,1 bis 3,0 Gew.-%, vorzugsweise 0,2 bis 2,5 Gew.-%, bezogen auf das Gesamtgewicht der Zusammensetzung, enthalten ist.

**Revendications**

1. Composition de conditionnement, qui est rincée et contenant

   - au moins un émollient choisi parmi des huiles d'ester et/ou un éther de dialkyle et/ou un carbonate de dialkyle,
   - au moins un émulsifiant
   - une combinaison d'alcools gras, dans laquelle un ou plusieurs alcools gras ayant 18 atomes de carbone ou plus sont contenus en une quantité totale inférieure à 1,5 % en poids et un ou plusieurs alcools gras ayant 16 atomes de carbone ou moins sont contenus en une quantité totale inférieure à 8,0 % en poids, respectivement chacune par rapport au poids total de la composition,
   dans laquelle l'au moins un émulsifiant est choisi parmi le stéarate citrate de glycéryle, le stéarate de glycéryle SE, le distéarate de polyglycéryle-3 en mélange avec le stéarate citrate de glycéryle et/ou le distéarate de polyglycéryle-3 méthyl glucose,
   dans laquelle l'au moins un émulsifiant est contenu en une quantité totale de 0,005 à 2,0 % en poids, par rapport au poids total de la composition,
   dans laquelle de l'eau est contenue en une quantité d'au moins 75 % en poids, de préférence de 85 à 98 % en poids d'eau, plus préférablement de 88 à 95 % en poids d'eau, par rapport au poids total de la composition et
   dans laquelle des polymères cationiques ne sont pas contenus.

2. Composition selon la revendication 1, **caractérisée en ce que** la composition est une émulsion, de préférence une émulsion H/E.

3. Composition selon la revendication 1 ou 2 **caractérisée en ce que** l'au moins un émulsifiant est un distéarate de polyglycéryle-3 en mélange avec du stéarate citrate de glycéryle.

4. Composition selon l'une quelconque des revendications précédentes, **caractérisée en ce que** de plus au moins un émulsifiant à base de silicone est contenu, préférablement du lauryl PEG/PPG 18/18 methicone.

5. Composition selon l'une quelconque des revendications précédentes, **caractérisée en ce que** l'au moins un émulsifiant est contenu en une quantité totale de 0,1 à 1,0 % en poids, par rapport au poids total de la composition.

6. Composition selon l'une quelconque des revendications précédentes, **caractérisée en ce que** l'au moins un émollient choisi parmi au moins une huile d'ester est choisi parmi le palmitate d'octyle, le cocoate d'octyle, l'isostéarate d'octyle, le myristate d'octyldodécyle, l'isononanoate de cétéaryle, le myristate d'isopropyle, le palmitate d'isopropyle, le stéarate d'isopropyle, l'oléate d'isopropyle, le stéarate de n-butyle, le laurate de n-hexyle, l'oléate de n-décyle, le stéarate d'isooctyle, le stéarate d'isononyle, l'isononanoate d'isononyle, le palmitate de 2-éthylhexyle, le laurate de 2-éthylhexyle, le stéarate de 2-hexyldécyle, le palmitate de 2-octyldodécyle, l'heptanoate de stéaryle, l'oléate d'oléyle, l'érucate d'oléyle, l'oléate d'érucyle, l'érucate d'érucyle, le stéarate de tridécyle, le trimellitate de tridécyle, le laurate d'isoamyle, de préférence parmi le myristate d'isopropyle et/ou le laurate d'isoamyle.

7. Composition selon la revendication 6, **caractérisée en ce que** l'au moins une huile d'ester est contenue en une quantité totale de 0,005 à 5,0 % en poids, de préférence de 0,1 à 3,0 % en poids, par rapport au poids total de la composition.

8. Composition selon l'une quelconque des revendications précédentes, **caractérisée en ce que** l'au moins un émollient est choisi parmi au moins un éther de dialkyle et/ou carbonate de dialkyle, de préférence parmi au moins un carbonate de dialkyle, plus préférablement le carbonate de dicaprylyle.

9. Composition selon la revendication 8, **caractérisée en ce que** l'au moins un éther de dialkyle et/ou carbonate de dialkyle est contenu en une quantité totale de 0,005 à 5,0 % en poids, de préférence de 0,1 à 3,0 % en poids, par rapport au poids total de la composition.

10. Composition selon l'une quelconque des revendications précédentes, **caractérisée en ce que** l'un du ou des alcools gras ayant 18 atomes de carbone ou plus est l'alcool stéarylique, de préférence l'alcool gras ayant 18 atomes de carbone ou plus est l'alcool stéarylique.

11. Composition selon l'une quelconque des revendications précédentes, **caractérisée en ce que** l'alcool myristylique et/ou l'alcool cétylique fait partie du groupe des alcools gras ayant 16 atomes de carbone ou moins, de préférence les alcools gras ayant 16 atomes de carbone ou moins sont l'alcool myristylique et l'alcool cétylique.

12. Composition selon l'une quelconque des revendications précédentes, **caractérisée en ce que** l'au moins un parmi l'alcool ou les alcools gras ayant 18 atomes de carbone ou plus est contenu en une quantité totale inférieure à 1 % en poids, par rapport au poids total de la composition.

13. Composition selon l'une quelconque des revendications précédentes, **caractérisée en ce que** l'au moins un parmi l'alcool ou les alcools gras ayant 18 atomes de carbone ou plus est contenu en une quantité totale supérieure à 0,1 % en poids, par rapport au poids total de la composition.

14. Composition selon l'une quelconque des revendications précédentes, **caractérisée en ce que** l'au moins un alcool(s) gras ayant 16 atomes de carbone ou moins est contenu en une quantité totale inférieure à 6,0 % en poids, de préférence inférieure à 5,0 % en poids, par rapport au poids total de la composition.

15. Composition selon l'une quelconque des revendications précédentes, **caractérisée en ce que** l'au moins un parmi l'alcool ou les alcools gras ayant 16 atomes de carbone ou moins est contenu en une quantité totale supérieure à 0,1 % en poids, par rapport au poids total de la composition.

16. Composition selon l'une quelconque des revendications précédentes, **caractérisée en ce que** de plus un ou plusieurs hydratants sont contenus, de préférence choisis parmi le propylène glycol, l'hexylène glycol et le butylène glycol, le triacétate de glycéryle, le glycérol, le sorbitol, le xylitol, le maltitol, le polydextrose, l'hydrolysat d'amidon hydrogéné, l'urée, le gel d'aloe vera, les alpha-hydroxyacides tels que l'acide lactique, le miel et/ou les polyéthylène glycols tels que le PEG-4, le PEG-6, le PEG-7, le PEG-8, le PEG-9, le PEG-10, le PEG-12, le PEG-14, le PEG-16, le PEG-18, le PEG-20, le PEG-32, le PEG-40, le PEG-55, le PEG-60, le PEG-75, le PEG-90, le PEG-100, le PEG-135, le PEG-150, le PEG-154, le PEG-180, le PEG-200, le PEG-240, plus préférablement choisis parmi la glycérine et PEG-150.

17. Composition selon la revendication 16, **caractérisée en ce que** l'au moins un hydratant est contenu en une quantité totale de 0,001 à 10 % en poids, de préférence de 0,005 à 6,0 % en poids, par rapport au poids total de la composition.

18. Composition selon l'une quelconque des revendications précédentes, **caractérisée en ce que** de plus au moins un composé cellulosique est contenu, de préférence au moins une hydroxy-alkylcellulose, plus préférablement au moins une hydroxyéthylcellulose.

19. Composition selon la revendication 18, **caractérisée en ce que** l'au moins un composé cellulosique est contenu en une quantité totale de 0,08 % à 1,0 % en poids, de préférence de 0,1 % à 0,6 % en poids, par rapport au poids total de la composition.

20. Composition selon l'une quelconque des revendications précédentes, **caractérisée en ce que** de plus au moins un tensioactif cationique est contenu, de préférence au moins un sel d'ammonium quaternaire, plus préférablement le chlorure de béhentrimonium.

21. Composition selon la revendication 20, **caractérisée en ce que** l'au moins un tensioactif cationique est contenu en une quantité totale de 0,1 à 3,0 % en poids, de préférence de 0,2 à 2,5 % en poids, par rapport au poids total de la composition.

**EP 4 065 237 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017036134 A1
- WO 2018018487 A1
- WO 2018227515 A1
- WO 2018095152 A1
- US 5139770 A
- EP 0937453 A2
- WO 2016090150 A1
- US 2009285869 A1